# EUROPEAN PATENT SPECIFICATION

(11) **EP 1 755 687 B1**
(45) Date of publication and mention of the grant of the patent: **09.11.2011**
(21) Application number: 04745172.9
(22) Date of filing: 15.06.2004
(51) Int. Cl.: A61L 2/10, C02F 1/32

(54) **IMPROVED APPARATUS FOR THE COLD STERILIZATION OF A FLUID BY ULTRAVIOLET RAYS**
VERBESSERTES GERÄT FÜR DIE KALTSTERILISATION EINER FLÜSSIGKEIT DURCH ULTRAVIOLETTE STRAHLEN
APPAREIL AMELIORE SERVANT A STERILISER A FROID UN FLUIDE PAR LES RAYONS ULTRAVIOLETS

(43) Date of publication of application: 28.02.2007
(73) Proprietor: S.I.D.E.A. Italia S.R.L., 50060 Molino del Piano, Pontasieve (IT)
(72) Inventor: TAVANTI, Giacomino, I-50100 Firenze (IT)
(74) Representative: Bardini, Marco Luigi
(86) International application number: PCT/IT2004/000346
(87) International publication number: WO 2005/123146

(56) References cited:
- EP-A- 0 378 716
- US-A- 4 968 437
- US-A- 5 874 741
- US-A1- 2002 096 648

## Description

### Field of the Invention

The present invention refers to the field of fluid sterilization and more precisely has as its object an improved apparatus for continuous cold sterilization of a fluid by ultraviolet rays.

### Description of the state of the art

In cold sterilization of fluids lamps are used arranged in the immediate vicinity of the fluid to be treated and capable of emitting ultraviolet wavelength radiation which, as known, has strong germicide properties.

In some types of cold sterilizing apparatuses, the lamps are sunk into the container with the fluid to be sterilized, whereas, in other types, the fluid passes through tubes, made of material permeable to ultraviolet radiation, which are exposed to the rays of the lamps arranged nearby. Amongst the known configurations of this second type of apparatus there are two that are more common having, in the first case, linear lamps surrounded by a bundle of tubes parallel to them and, in a second case, many parallel lamps arranged between two curtains of parallel tubes.

The results that can be obtained with cold sterilization through ultraviolet rays is all the better the longer the exposure of the fluid to the action of the ultraviolet radiation and the more the material of the tubes in which the fluid circulates is permeable to this radiation. Nevertheless, in the two configurations of the second type of apparatus described above, to obtain a treatment path of sufficient length, it is necessary to arrange a large number of lamps in series giving rise to a correspondingly bulk apparatus or, alternatively, it is necessary to excessively slow down the speed of the fluid in the transparent tubes creating problems of insufficient capacity of the apparatus in the case in which high fluid flow rates must be treated.

Again in this type of cold sterilizer, with fluid that flows in transparent tubes and that is exposed to ultraviolet radiation, there are also another two drawbacks. The first is the incomplete exploitation of the radiation that, filtering through the tubes and the fluid, dissipates on the inner walls of the apparatus. The second is represented by the risk of incomplete or even inexistent sterilization in the case in which the lamp is switched off or has run out.

An apparatus for the continuous cold sterilization of a fluid that overcomes the aforementioned drawbacks has been produced and commercialised by the same Applicant. In this apparatus a duct transparent to ultraviolet radiation and extending helically around the ultraviolet radiation source has been provided. A tubular screen is arranged outside of and coaxially to the helical duct. This apparatus, whilst operating satisfactorily, has the drawback of excessively heating the water mainly when there is a low water demand and when the lamp has been switched on for a long time. In these circumstances, the water, staying still for a long time in the helical duct, can immediately be used only at a substantially higher temperature than room temperature and can also reach temperature levels unsuitable for the apparatus and for the user.

US-A-2002/0096648 discloses an apparatus for the continuous cold sterilization of fluids of similar type in which the duct may have an oval cross section. US-A-4968437 discloses a fluid purification system of similar type comprising light means for controlling that the system is turned on and properly powered.

### Objects and summary of the invention

The objects of the present invention is to provide an apparatus for the continuous cold sterilization of a fluid that allows the aforementioned problems to be solved and more specifically that does not have the drawback of the heating of the fluid without however penalizing the exploitation of the ultraviolet radiation emitted by the lamp and therefore the sterilizing capability of the apparatus.

This and other objects are accomplished with the continuous cold sterilization apparatus according to the present invention, the essential features of which are set forth in the attached claim 1.

### Brief description of the drawings

Further characteristics and advantages of the apparatus for the cold sterilization of a fluid by ultraviolet rays according to the present invention shall become clearer from the following description of an embodiment thereof, given as a non-limiting example, with reference to the attached drawing, in which:
- figure 1 is a sectional view of the apparatus made according to a vertical plane passing through the axis of the lamp;
- figure 2 is an enlarged view of the detail indicated with A in figure 1.
- figure 3 is a side view of the apparatus.

### Detailed description of the invention

With reference to the aforementioned figures, an apparatus for continuously cold sterilizing a fluid comprises a box-shaped casing 1, at least one ultraviolet radiation source 2, of the linear tubular lamp type, and at least one duct 3 permeable to such radiation in which a fluid to be sterilized 4 flows. The duct 3 has a portion 3a that extends helically around the source 2, so that all of the radiation emitted by it crosses the fluid 4 and exerts the germicide action. At its outlet the duct 3 is connected to a dispensing device 10.

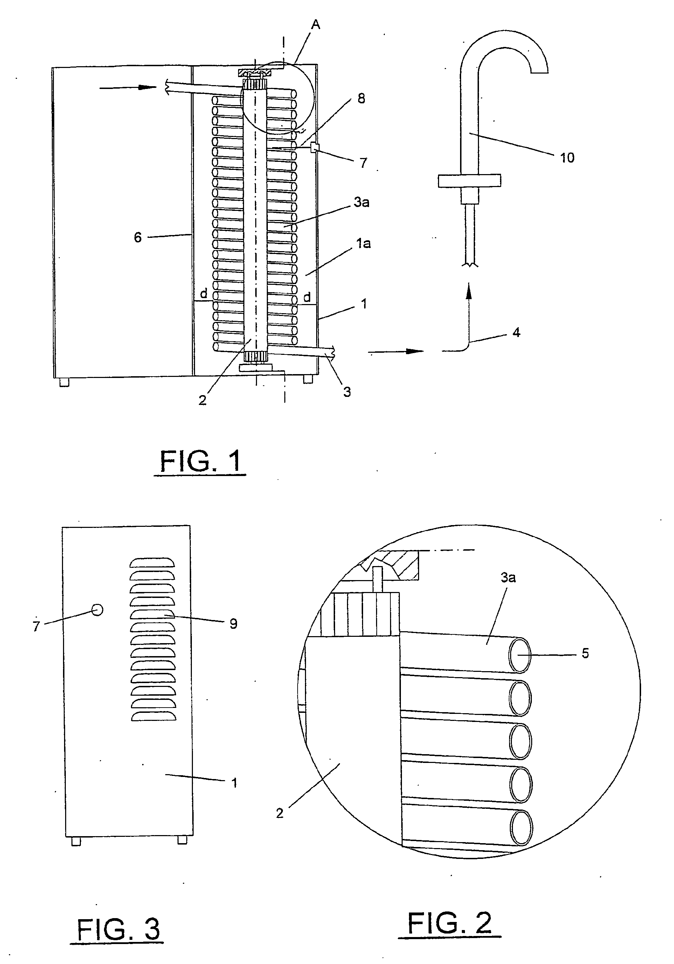

According to the invention, the helical portion 3a of the duct 3 is arranged in a chamber 1a of the box-shaped casing 1 defined by the walls of the casing 1 and by an inner wall 6. The inner surface of said walls has good characteristics of reflection to ultraviolet radiation, said walls preferably being made from stainless steel. In this way the residual ultraviolet radiation, passing through the helical portion 3a and the fluid that flows in it, is reflected onto the fluid to be treated. To avoid the heating of the water during some steps of the sterilization process, the helical portion 3a of the duct 3 is suitably spaced from the wall 6 and from the outer wall of the casing 1, such a distance, indicated with d in figure 1, being at least equal to 5 mm and preferably equal to 15 mm.

Thanks to the teaching of suitably spacing the walls of the chamber la from the tubular portion 3a, the air circulation and heat exchange is promoted. Also contributing to achieving such a result is the presence of slits 9, shown in figure 3, formed on the walls of the chamber 1a that allow the circulation of air between the chamber 1a and the outside.

In order to avoid a loss of treatment efficiency due to the distance between the reflective surfaces and the helical portion 3a, the latter has an elliptical-shaped passage section 5 for the fluid with its major axis parallel to the axis of the tubular light source 2 or, in general, perpendicular to the irradiation direction. The squashing of the section of the tube forming the tubular portion 3a significantly decreases the thickness of the fluid that the light radiation must cross thus realising a significant gain in efficiency somewhat greater than the loss of efficiency due to the distancing of the reflective surface.

The control of the operation of the apparatus and in particular of the irradiation conditions is carried out through an indicator light 7 fixed to the outside of the casing 1 and communicating optically with the lamp 2 by means of an optical fibre 8 positioned in contact with the lamp or in its immediate vicinity. By checking the operation through the indicator light 7 it is possible to find out whether the apparatus is working correctly and in particular to prevent drawbacks like the switching off or the running out of the lamp that can impair the quality of sterility of the outlet fluid 4.

Advantageously, the duct 3, or at least its helical portion 3a, is made from teflon or equivalent materials.

The apparatus according to the invention fully achieves the predetermined purposes. In particular, it allows a fluid in transit through the duct 3 to be sterilized increasing the efficiency with respect to the previously known solution and keeping the temperature at completely acceptable values, in any case never greater than those reached by the lamp 2, even in the most extreme operating conditions quoted previously.

Variations and/or modifications may be brought to the apparatus for continuously cold sterilizing a fluid according to the present invention, without departing from the scope of the invention as defined in the attached claims.

## Claims

1. Apparatus for the continuous cold sterilization of a fluid comprising at least one ultraviolet radiation source (2) and at least one duct (3) permeable to such radiation in which said fluid flows, said duct having a portion (3a) that extends helically around said source, said helical portion (3a) being arranged in a chamber (1a) the walls of which have reflective surfaces, the distance (d) between said walls and said helical portion being sufficient to allow the circulation of air between them, said helical portion of the duct for the fluid having an elliptical-shaped passage section (5) with the major axis perpendicular to the irradiation direction, the apparatus being **characterized in that** air circulation slits (9) are formed on the walls of said chamber.

2. Apparatus according to claim 1, wherein the distance between the walls of said chamber (1a) and said helical portion (3a) is at least 5 mm.

3. Apparatus according to any one of the previous claims, wherein said ultraviolet radiation source (2) is tubular-shaped and the major axis of the elliptical section of said helical portion is parallel to the longitudinal axis of said source.

4. Apparatus according to any one of the previous claims, wherein outside of said chamber (3a) an indicator light (7) is provided and is optically connected to said ultraviolet radiation source (2) through an optical fibre (8) placed in contact with the lamp or in its immediate vicinity.

## Patentansprüche

1. Gerät für die kontinuierliche Kaltsterilisation eines Fluides, enthaltend wenigstens eine ultraviolette Strahlungsquelle (2) und wenigstens eine für eine derartige Strahlung durchlässige Leitung (3), in der das Fluid fließt, wobei die Leitung einen Teil (3a) hat, der spiralförmig um die Quelle herum verläuft, wobei der spiralförmige Teil (3a) in einer Kammer (1a) angeordnet ist, deren Wände reflektierende Oberflächen haben, wobei der Abstand (d) zwischen den Wänden und dem spiralförmigen Teil ausreichend ist, um die Zirkulation von Luft zwischen ihnen zu gestatten, wobei der spiralförmige Teil der Leitung für das Fluid eine elliptisch geformte Durchgangssektion (5) mit der Hauptachse senkrecht zu der Bestrahlungsrichtung hat, wobei das Gerät **dadurch gekennzeichnet ist, dass** Luftzirkulationsschlitze (9) in den Wänden der Kammer ausgebildet sind.

2. Gerät nach Anspruch 1, wobei der Abstand zwischen den Wänden der Kammer (1a) und dem spiralförmigen Teil (3a) wenigstens 5mm ist.

3. Gerät nach einem der vorhergehenden Ansprüche, wobei die ultraviolette Strahlungsquelle (2) rohrförmig ist und die Hauptachse der elliptischen Sektion des spiralförmigen Teils parallel zu der Längsachse der Quelle ist.

4. Gerät nach einem der vorhergehenden Ansprüche, wobei außerhalb der Kammer (1a) ein Indikatorlicht vorgesehen ist und optisch mit der ultravioletten Strahlungsquelle (2) durch eine optische Faser (8) verbunden ist, die in Kontakt mit der Lampe oder ihrer unmittelbaren Umgebung platziert ist.

## Revendications

1. Appareil pour la stérilisation à froid continue d'un fluide comprenant au moins une source de rayonnement ultraviolet (2) et au moins un conduit (3) perméable à un tel rayonnement dans lequel s'écoule ledit fluide, ledit conduit ayant une partie (3a) qui s'enroulant de façon hélicoïdale autour de ladite source, ladite partie hélicoïdale (3a) étant agencée dans une chambre (1a) dont les parois comportent des surfaces réfléchissantes, la distance (d) entre lesdites parois et ladite partie hélicoïdale étant suffisante pour permettre la circulation d'air entre elles, ladite partie hélicoïdale du conduit pour le fluide ayant une section de passage (5) de forme elliptique avec l'axe principal perpendiculaire à la direction de rayonnement, l'appareil étant **caractérisé en ce que** des fentes (9) de circulation d'air sont réalisées dans les parois de ladite chambre.

2. Appareil selon la revendication 1, où la distance entre les parois de ladite chambre (1a) et ladite partie hélicoïdale (3a) est d'au moins 5 millimètres.

3. Appareil selon l'une quelconque des revendications précédentes, où ladite source de rayonnement ultraviolet (2) est de forme tubulaire et l'axe principal de la section elliptique de ladite partie hélicoïdale est parallèle à l'axe longitudinal de ladite source.

4. Appareil selon l'une quelconque des revendications précédentes, où est prévue une lampe témoin en dehors de ladite chambre (1a), ladite lampe témoin étant optiquement couplée à ladite source de rayonnement ultraviolet (2) au moyen d'une fibre optique (8) placée au contact de cette source ou à proximité immédiate.
